# FASCICULE DE BREVET EUROPEEN

(11) **EP 1 468 277 B1**
(45) Date de publication et mention de la délivrance du brevet: **04.07.2007**
(21) Numéro de dépôt: 02799807.9
(22) Date de dépôt: 11.12.2002
(51) Int. Cl.: C12Q 1/00

(54) **DETECTION DE MOLECULES SONDES FIXEES SUR UNE ZONE ACTIVE D'UN CAPTEUR**
NACHWEIS VON SONDENMOLEKÜLEN, DIE AUF EINEN AKTIVEN BEREICH EINES SENSORS GEBUNDEN SIND
DETECTION OF MOLECULAR PROBES FIXED TO AN ACTIVE ZONE OF A SENSOR

(30) Priorité: 21.01.2002 FR 0200676
(43) Date de publication de la demande: 20.10.2004
(73) Titulaire: CENTRE NATIONAL DE LA RECHERCHE SCIENTIFIQUE (CNRS), 75794 Paris Cedex 16 (FR)
(72) Inventeur: BOCKELMANN, Ulrich, F-75020 Paris (FR); POUTHAS, François, F-75013 Paris (FR)
(74) Mandataire: Jacquard, Philippe Jean-Luc
(86) Numéro de dépôt international: PCT/FR2002/004283
(87) Numéro de publication internationale: WO 2003/062811

(56) Documents cités:
- EP-A- 1 460 130
- US-A- 4 238 757
- US-A- 5 827 482
- US-B1- 6 322 963
- US-B1- 6 331 274

## Description

La présente invention a pour objet un procédé de détection d'au moins un paramètre représentatif de molécules sondes fixées sur des zones d'un capteur.

On connaît déjà un procédé de détection de l'hybridation de séquences d'ADN à l'aide d'un transistor à effet de champ, tel qu'il a été décrit dans l'article de E. SOUTEYRAND et collaborateurs intitulé « Direct Detection of the hybridization of synthetic Homo-Oligomer DNA Sequences by Field Effect » paru en 1997 dans le J. Phys. Chem. B1997, 101, pages 2980 à 2985. Un transistor de type ISFET (« Ion-Sensitive - Field Effect Transistor ») utilisable dans ce type d'application a été décrit dans l'article de Piet BERGVELD « Development, Operation and Application of the ISFET as a Tool for Electrophysiology paru dans IEEE Transactions on Biomedical Engineering volume BME-19- n° 5 Sept 1972 pages 342 à 351. Des indications sur la fabrication de telles structures de transistors peuvent être trouvées dans l'article de V. KIESSLING et collaborateurs, intitulé « Extracellular Resistance in Cell Adhesion Measured with a Transistor Probe » paru dans Langmuir 2000 16, pages 3517 - 3521. Enfin un mode de préparation des surfaces a été décrite dans l'article de A. KUMAR et collaborateurs, intitulé « Silanized nucleid acids : a general platform for DNA immobilization » paru dans Nucleid Acid Research 2000, volume 28, n° 14, pages i à vi.

Deux procédés de fixation des molécules sondes sur la surface sont notamment utilisables dans le cadre de la présente invention. Le premier consiste en une synthèse directe sur solide, telle que décrite par exemple dans l'article de S.P.A. Fodor et Collaborateurs intitulé « Light-directed, spatially adressable parallel chemical synthesis » paru dans Science 251, pages 767 à 773 (1991). Le deuxième est la fixation des molécules à partir d'une dilution.

Dans le cas des capteurs comportant une pluralité de zones actives, par exemple les puces à ADN, ou les puces à protéines, il n'existe pas actuellement de technique disponible permettant de contrôler facilement et de la manière relativement rapide sur quelles zones des molécules sondes ont été fixées effectivement.

Les Brevets US 5,827,482 et US 6,331,274 de même que la Demande de Brevet EP 1 460 130 publiée le 26 Juin 2003 concernent des procédés de détection de la fixation de molécules sur des zones actives en utilisant un réseau de transistors à effet de champ. Dans ces documents, il n'est pas prévu de fixer le potentiel de la solution qui recouvre les zones actives.

La présente invention a ainsi pour but un procédé de détection de la fixation de molécules sondes sur au moins une zone d'un capteur, ou d'une interaction, notamment en vue de contrôler le dépôt et la fixation locales des molécules sondes, pour permettre en particulier de remédier au moins partiellement aux problèmes que posent les variations expérimentales importantes qui sont fréquemment rencontrées en pratique.

La présente invention concerne ainsi un procédé de détection de la fixation de molécules sondes sur au moins une zone active d'un capteur ou d'une interaction, tel que défini dans la revendication 1.

Entre les étapes a et b, il peut être prévu un rinçage.

Selon un mode particulier de mise en oeuvre, le procédé est caractérisé en ce qu'il comporte après a) et avant b) les étapes suivantes :
a1) rinçage
a2) ajout d'une solution contenant des molécules cibles susceptibles d'interagir spécifiquement avec les molécules sondes, par exemple de s'hybrider avec celles-ci dans le cas où les molécules sondes sont de l'ADN, éventuellement suivi d'un rinçage.

Selon un autre mode particulier de mise en oeuvre, le procédé est caractérisé en ce qu'il comporte après c, les étapes suivantes :
d) ajout d'une solution d'électrolyte contenant des molécules cibles susceptibles d'interagir spécifiquement avec les molécules sondes, par exemple de s'hybrider dans le cas où les molécules sondes sont de l'ADN.
e) mesurer au moins un point de la caractéristique courant de drain/tension source-porte / tension source-drain d'au moins deux des transistors d'un deuxième groupe correspondant à des zones mises en contact avec des molécules sondes et avec des molécules cibles, par exemple en appliquant une tension par exemple constante entre la porte et la source de ces transistors du deuxième groupe dont le drain et la source ont été polarisés ou un courant donné par exemple constant à la source de ces transistors du deuxième groupe, pour obtenir par comparaison une détection d'au moins une interaction.

Le procédé peut mettre en oeuvre une pluralité de dites mesures d'au moins un point de la caractéristique, qui sont espacées au cours du temps. Ceci permet une mesure doublement comparative, dans l'espace et dans le temps.

Selon une première variante, la comparaison, notamment par mesure différentielle, est réalisée entre au moins deux transistors correspondant à des zones qui sont baignées par une solution d'électrolyte, après avoir été mises en contact avec des molécules sondes.

Selon une deuxième variante préférée, cette comparaison, notamment par mesure différentielle, est réalisée entre au moins un transistor correspondant à une zone qui est baignée par une dite solution d'électrolyte après avoir été mise en contact avec des molécules sondes et au moins un transistor correspondant à une zone qui est baignée par ladite solution d'électrolyte sans avoir été préalablement mise en contact avec des molécules sondes.

Les molécules sondes sont par exemple des molécules d'ADN, d'ARN ou de protéines.

Le procédé selon l'invention est compatible avec une détection classique d'interaction moléculaire par fluorescence.

D'autres caractéristiques et avantages de l'invention apparaîtront mieux à la lecture de la description ci-après, en liaison avec les dessins annexés dans lesquels :
- la figure 1 représente deux transistors à effet de champ d'une puce de détection comprenant une pluralité de tels transistors organisés suivant un réseau mono ou bidimensionnel de transistors ;
- la figure 2 représente en vue de dessus un détail d'une puce de détection et l'agencement des zones actives correspondant chacune à un transistor à effet de champ ;
- la figure 3 illustre les connexions électriques de drain des transmissions du réseau mono ou bidimensionnel, la figure 4 représentant la résistance des différentes connexions électriques de drain, la courbe A représentant les valeurs calculées et la courbe B les valeurs mesurées, l'écart entre les courbes étant dû pour l'essentiel à la résistance de canal qui est constante.
- la figure 5 représente un dispositif de dépose de la solution sur des zones actives sélectionnées ;
- la figure 6 illustre la détection de présence d'ADN silanisé et de poly-L-lysine avec U_{SG} et U_{SD} constants, par variation du courant de drain I_{SD} ; et
- la figure 7 illustre la détection de présence d'ADN silanisé et de poly-L-lysine avec I_{SD} et U_{SD} constants, par détection de la variation de la tension U_{SG} ;
- les figures 8A à 8C représentent les résultats d'expériences réalisées dans différentes conditions expérimentales ;
- les figures 9A à 9D montrent une détection électronique d'ADN ;
- et les figures 10A et 10B illustrent la mise en oeuvre de canaux microfluidiques.

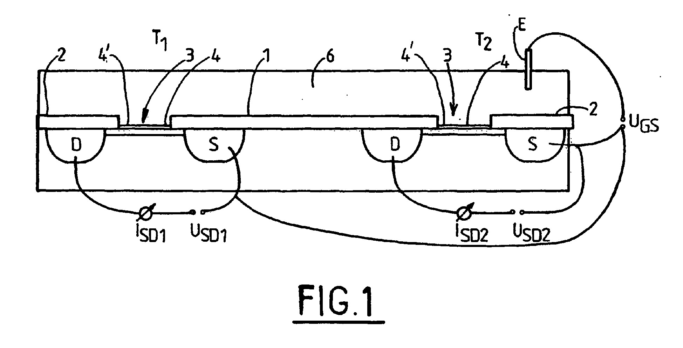

Les figures 1 à 3 illustrent un capteur présentant un réseau de transistors à effet de champ sur un substrat en silicium. Un transistor T₁ ou T₂ représenté en coupe à la figure 1 est pourvu d'une région de source S et d'une région de drain D qui présentent chacune un contact électrique et qui sont surmontées d'une couche isolante respectivement 1 et 2, par exemple un oxyde thermique de SiO₂. La région active 3 entre la source S et le drain D forme la région de porte G du transistor et présente une couche isolante 4 d'épaisseur réduite, par exemple une couche de SiO₂ thermique. Il est également possible de ne pas disposer d'oxyde sur cette région active. La surface active est alors délimitée par une portion 4' du substrat qui est mise à nu.

Des molécules sondes, par exemple des molécules d'ADN simple brin, sont fixées par un procédé connu sur au moins certaines des surfaces actives 4 ou 4'. Pour l'ADN, on utilise de préférence des transistors à effet de champ à canal n à appauvrissement (pour lesquels les porteurs de charge sont les électrons, plus mobiles d'où une augmentation de la sensibilité) avec une polarisation de porte négative (c'est-à-dire que l'électrolyte est polarisé négativement par rapport au semi-conducteur), l'ADN se chargeant négativement (pour un électrolyte de pH neutre).

L'application d'une tension source-drain U_{SD} entre la source S et le drain D (U_{SD1}, pour T₁, et U_{SD2} pour T₂) et d'une tension porte-source U_{GS} entre l'électrolyte 6 et la source S (par exemple par une électrode E unique Ag/AgCl) induit un gaz bidimensionnel de porteurs de charges à l'interface Si/SiO₂, ou à l'interface Si/électrolyte de chaque transistor. Il en résulte un courant de drain I_{D} qui, pour chaque transistor, dépend de façon sensible de la charge à l'interface SiO₂/électrolyte ou Si/électrolyte. On appelle surface active cette interface qui fait face au canal entre la source S et le drain D.

Le courant I_{D} dépend de la fixation des molécules sondes, par exemple des molécules d'ADN sur la surface active 4 ou 4'.

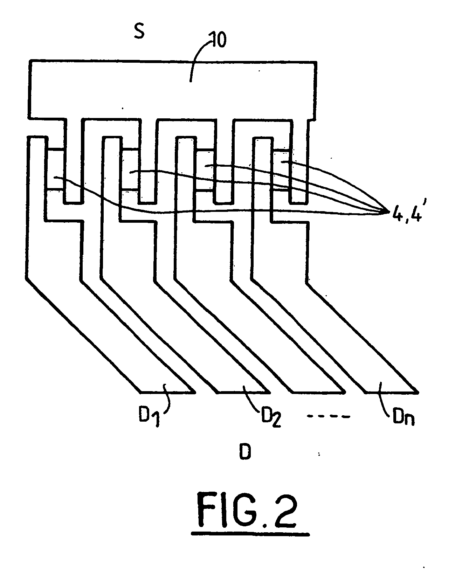

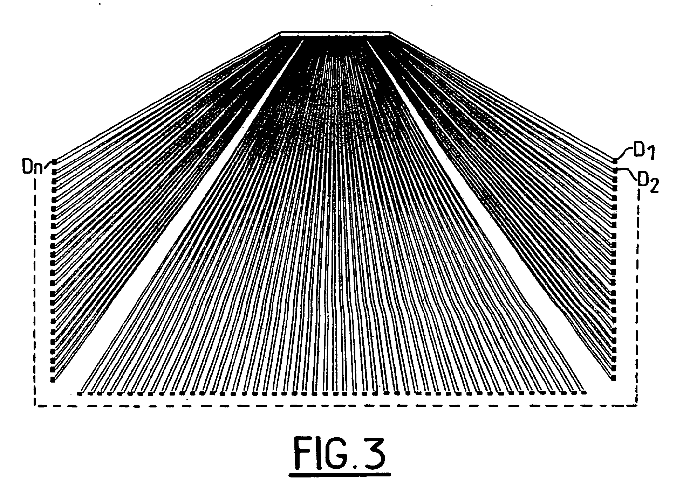

Comme le montrent les figures 2 et 3, n structures de type transistor à effet de champ sont intégrées dans un substrat de silicium recouvert d'un isolant (SiO₂ ou autre) et pourvu de connexions appropriées (métallisation ou de préférence régions conductrices dopées) par les prises de contact électrique de source 10 et de drain (D₁, ... Dₙ). A la différence d'une structure de transistor MOS standard, il n'y a pas d'électrode métallique de porte. Ceci correspond à la structure de type « ISFET » (« Ion Sensitive Field Effect Transistor »). On utilise de préférence un substrat de type SOI (silicium sur isolant) qui procure une sensibilité plus élevée.

Les différentes structures sont à faible distance latérale l'une de l'autre et leurs surfaces actives sont en contact avec la même solution de mesure. Une dimension latérale typique dans la micro-électronique actuelle est inférieure au µm. Dans la technologie des puces à ADN telle que mise en oeuvre dans la présente invention, la dimension latérale est de 5-10 µm pour une synthèse directe sur le solide et de 50-100 µm dans le cas d'une fixation des molécules à partir d'une dilution.

Dans la présente configuration de mesure parallèle, plusieurs plots avec différents types de molécules sondes immobilisées sont en contact avec la même solution de mesure et au moins une structure de transistor se situe en dessous de chaque plot. La mise en oeuvre de plusieurs transistors par plot est possible au vu des dimensions mentionnées ci-dessus et permet une redondance dans la détection.

Une électrode E (Ag/AgCl, par exemple), est utilisée pour fixer le potentiel de la solution de mesure 6 (électrolyte) par rapport à la structure en silicium qu'elle recouvre et pour fixer le point de travail des capteurs (transistors). Le potentiel de l'électrolyte 6 peut dans certains cas être égal à zéro. La solution de mesure 6 qui baigne les capteurs contient des ions à une concentration qui donne une conductivité suffisante et qui ne donne pas lieu à un écrantage trop important au niveau des surfaces actives. Son pH est de préférence neutre.

Le procédé de détection des reconnaissances moléculaires est basé sur une approche par comparaison, notamment différentielle. La mesure est réalisée en utilisant plusieurs structures de transistor en parallèle. La mesure peut être différentielle par rapport aux différents types de molécules greffées et inclure éventuellement plusieurs transistors par type de molécule. Il est également possible de comparer des signaux avant/après la réaction qui révèle la reconnaissance moléculaire (et/ou l'évolution pendant cette réaction).

Le procédé selon l'invention permet de contourner les difficultés associées à la sensibilité d'un capteur individuel au pH et à la force ionique et celles associées à une variabilité d'un transistor individuel à l'autre (ceci inclut la structure de transistor et la qualité de la fixation des sondes).

Un procédé selon un mode de réalisation préféré met en oeuvre les étapes suivantes :
a) traitements homogènes de toute la surface isolante pour préparer la fixation des molécules sondes ;
b) greffage local de différents types de molécules sondes sur au moins certaines des surfaces actives individuelles ;
c) rinçages homogènes;
d) mesure électronique : ajouter l'électrolyte de mesure, plonger l'électrode et mesurer les transistors (par exemple un ou plusieurs points de la caractéristique I_{D} en fonction de U_{SD} et de U_{SG}), et comparer les résultats obtenus selon les transistors ;
e) rinçages homogènes ;
f) et éventuellement ajout de la solution de molécules cibles en présence d'électrolyte et réaction de reconnaissance;
g) rinçages homogènes ;
h) mesure électronique, comme (d).

En cas de mise en oeuvre des étapes f à h, il est possible d'omettre c et d, c'est-à-dire de ne faire qu'une mesure électronique.

Certains transistors qui n'ont pas été mis en présence de molécules sondes (ou bien un seul transistor) peuvent servir de témoins. On mesure leurs caractéristiques après ajout de l'électrolyte de mesure qui par exemple, baigne l'ensemble des transistors.

Le greffage des molécules sondes est réalisé par dépôt de micro-gouttelettes de diamètre environ 100 µ sur les surfaces actives des transistors à l'aide de micro-plumes métalliques qui sont disponibles commercialement.

Comme le montre la figure 3, le réseau de n transistors (par exemple n = 96 transistors) présente n connexions de drain D₁, D₂ ... Dₙ et 2 connexions (non représentées) équivalentes à la source commune. Les résistances série R_{c} associées à ces connexions ont des valeurs qui dépendent de l'index 1 ...n du drain.

Les valeurs de ces résistances R_{c}, réalisées par exemple par dopage du silicium, ne sont pas négligeables.

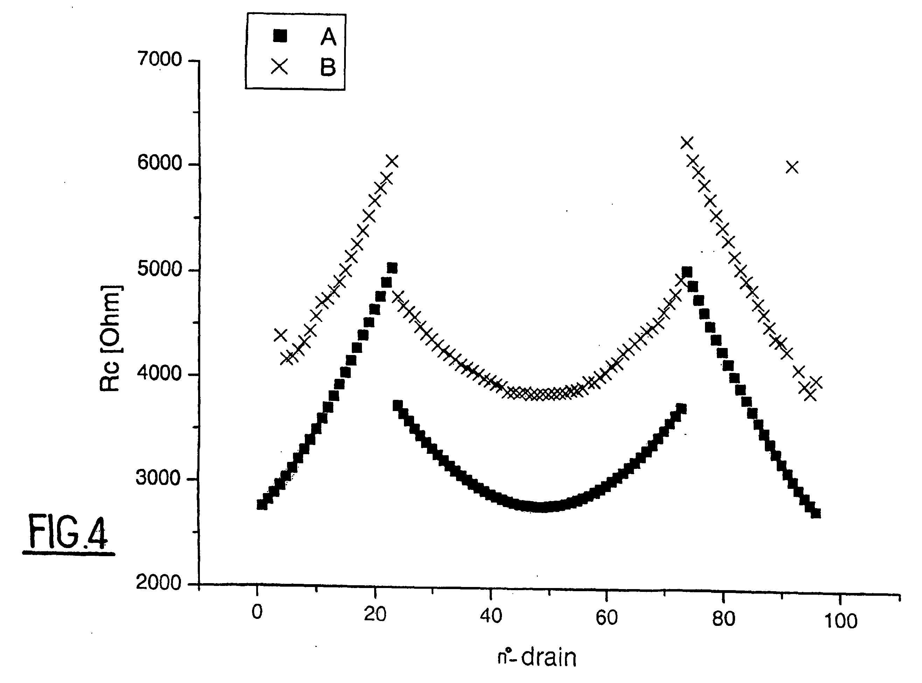

A cet effet les résistances Rc de connexion de drain sont calculées à partir des longueurs et des sections géométriques des lignes dopées dont on connaît la résistivité. Le calcul est comparé à une mesure de la résistance en fonction de l'index du drain en appliquant une tension continue (par exemple U_{SD} = 0,1V et U_{SG} = 2V). Ceci permet d'obtenir une courbe de compensation donnée à titre d'exemple à la figure 4.

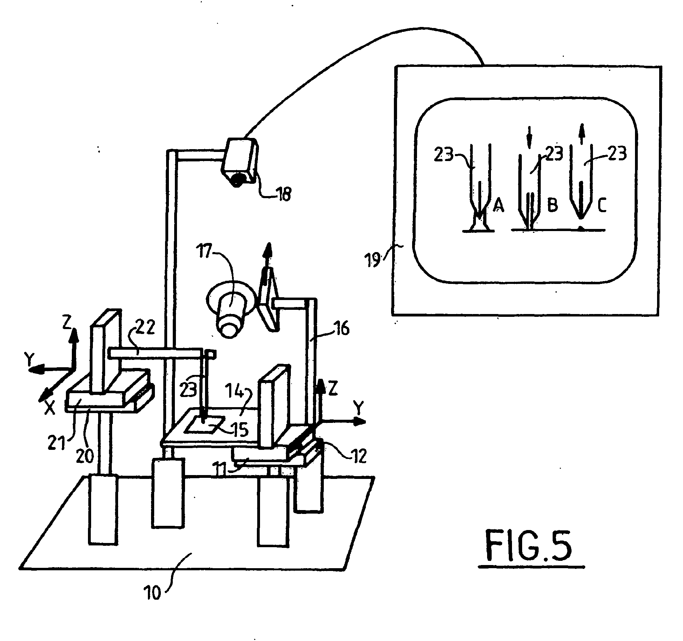

Une installation telle que celle représentée à la figure 5 peut être utilisée pour la mise en oeuvre du procédé : sur une table 10, est disposée une platine 12 incorporant un dispositif de commande à micro-contrôleur pour une table 11 assurant un déplacement selon trois directions perpendiculaires X, Y, Z. Une puce 15 incorporant le réseau de n transistors est disposée sur un support 14. Une autre platine 20 comportant une table 21 assurant un déplacement selon les trois directions X, Y, et Z est mise en oeuvre pour déplacer un bras 22 portant une micro-plume ou une pipette 23 pour assurer le dépôt des micro-gouttelettes sur au moins certains des n transistors. Un objectif 17 et/ou une caméra couplée à un écran 19 permettent d'observer le dépôt des micro-gouttelettes et de contrôler les opérations.

On effectue des mesures de courant de drain I_{D} avec par exemple U_{SG} = 1V et U_{SD} = 0,9V et un électrolyte de pH neutre déposé qui est constitué de KCl à une teneur de 0,1 millimole par litre. Les transistors (canal p à accumulation) ayant leurs sources interconnectées, la tension de source ou la tension de porte peut servir de référence de tension (par exemple la tension de masse).

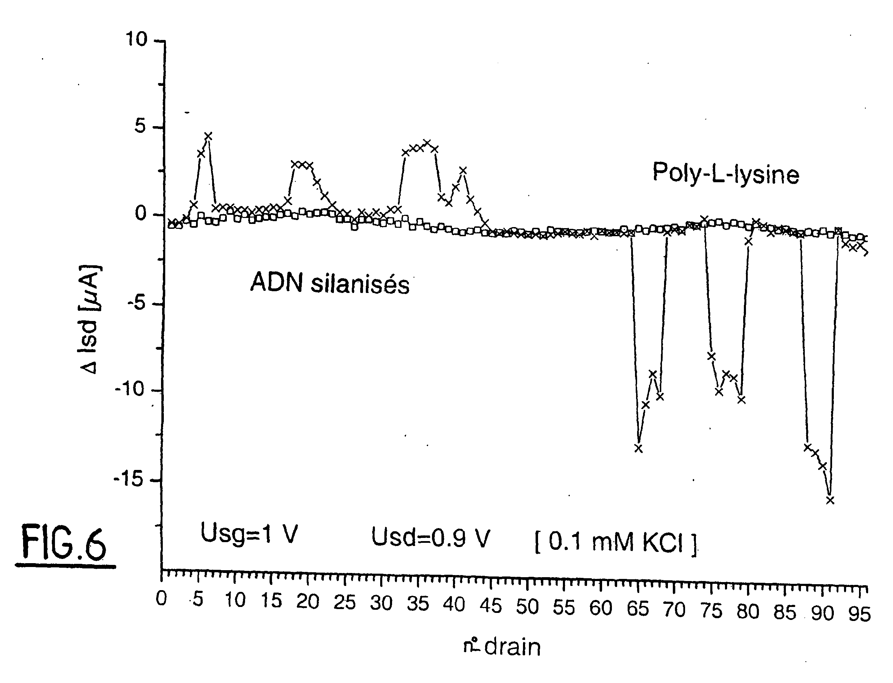

Une mise en oeuvre du procédé sera maintenant décrite en liaison avec la figure 6.

Avant ces mesures, on effectue un traitement global de la surface de la structure Si/SiO₂ par incubation 1-2 minutes dans de l'acide sulfochromique et rinçage sous un courant d'eau déionisée puis incubation 3 à 5 minutes dans une solution de NaOH (60 µl NaOH 16N, 420µl d'éthanol et 220 µl d'eau), et enfin rinçage sous un courant d'eau déionisée.

La différence entre deux mesures effectuées avant dépôt local mais avant et après un rinçage à l'eau est présentée en petits carrés à la figure 6. Les croix représentent la différence entre une mesure effectuée après dépôt local de deux solutions différentes et une mesure faite avant le dépôt (la mesure faite avant le rinçage à l'eau).

Avec une pointe commerciale 23 (Telechem SMP3B) montée sur le dispositif 22 présenté à la figure 5, on dépose une solution 1 sur les transistors 5-7 (avec un contact entre pointe et surface), les transistors 19-21 et les transistors 33-37 et une solution 2 sur les transistors 66-69, les transistors 76-79, et les transistors 87-89.

Solution 1 : 0,5 µl oligonucléotide 20 mer modifiée thiol en 5' à 1 nmol/µl, 9 µl sodium acétate 30 mM à pH 4,3, 0,5 µl mercaptosilane 5mM dans sodium acétate, que l'on laisse réagir une heure à température ambiante, avant le dépôt.

Solution 2 : Poly-L-lysine (0,01% poids/volume « w/v » final (P8920, Sigma)) dans un tampon PBS 0,1X à pH 7.

Après les dépôts locaux, l'échantillon est séché 15 minutes en atmosphère humide et ensuite 5 minutes à 50°C.

La poly-L-lysine est positive dans l'électrolyte de mesure (pH neutre) à cause des groupements amines ionisés. La diminution du courant observé sur les dépôts de poly-L-lysine est compatible avec l'adsorption d'une charge positive sur la surface.

Pour la solution 1, la modification silane sur l'ADN réagit avec les groupements OH de SiO₂ et l'ADN est chargé négativement en solution.

Les solutions 1 et 2 donnent donc des signaux de signes opposés.

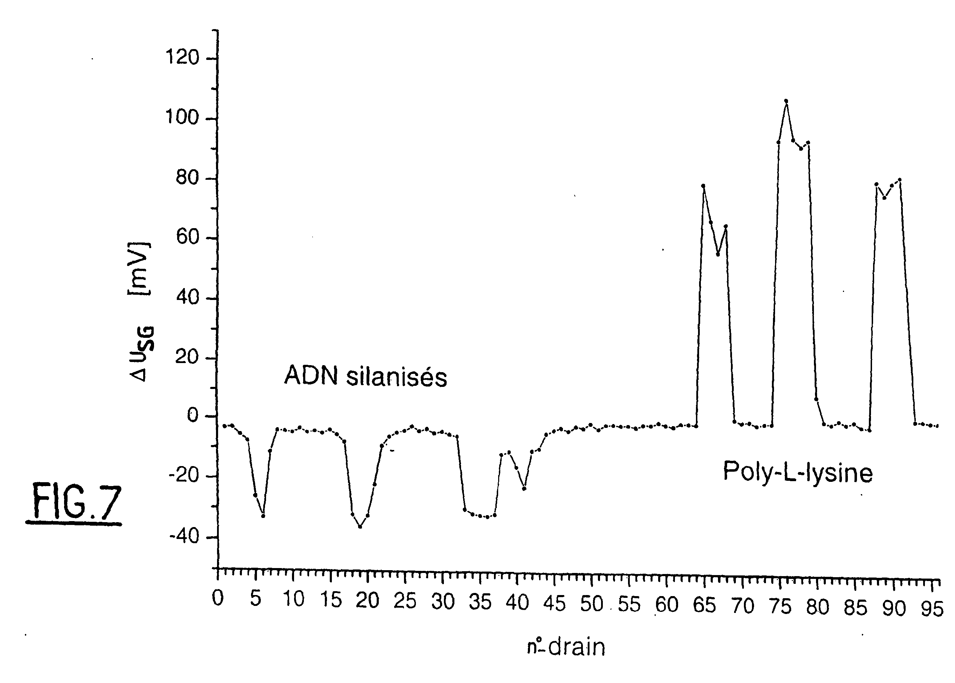

Une autre mise en oeuvre de procédé sera maintenant décrite en liaison avec la figure 7.

On mesure la différence de potentiel de surface ΔU_{SG} correspondant à la mesure avant/après dépôt. Pour déterminer ΔU_{SG}, on mesure la caractéristique bidimensionnelle par exemple I_{D}(U_{SG}, U_{SD}) et on détermine les caractéristiques intrinsèques des 96 transistors en corrigeant numériquement en fonction des résistances R_{c} des lignes de drain en série. La modification de l'état de l'interface SiO₂ induit un changement de la caractéristique intrinsèque qui correspond à un décalage ΔU_{SG} à U_{SD} et courant de drain I_{D} constants. Ce décalage permet d'obtenir directement une mesure indépendante du point de travail du transistor, contrairement au changement de courant ΔI_{D} présenté dans la figure 6. La valeur ΔU_{SG} permet en première approximation de quantifier le changement de l'interface SᵢO₂/liquide induit par le dépôt local. Selon une variante, on fait varier U_{SG} de façon à garder I_{D} constant.

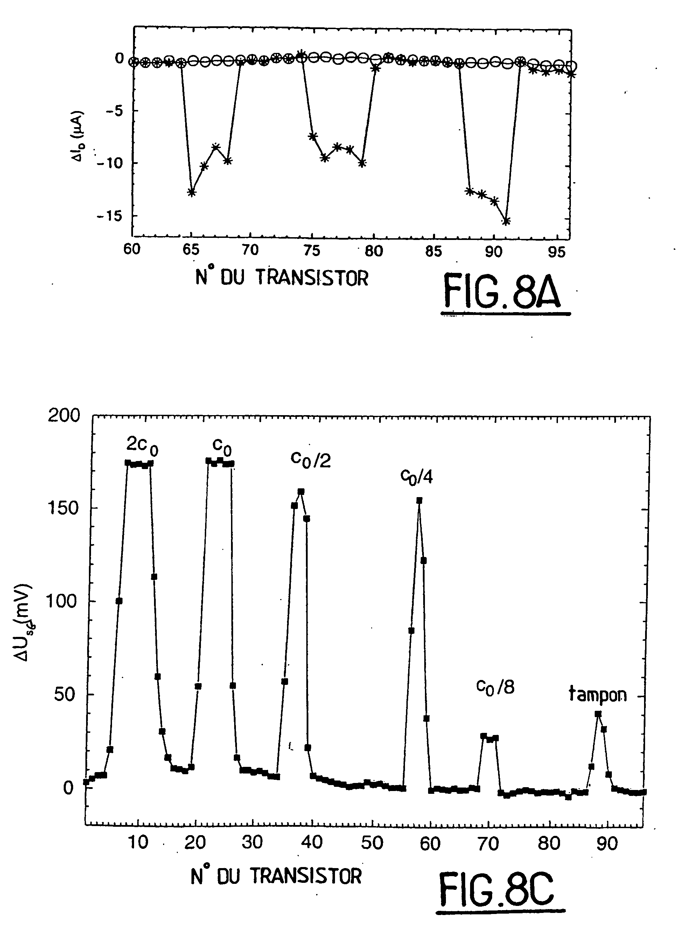

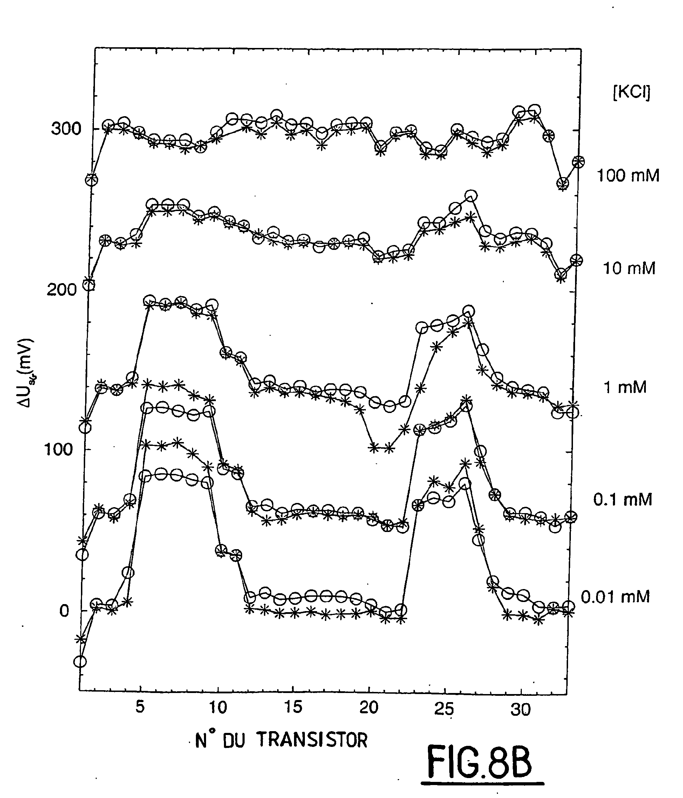

Les figures 8A à 8C montrent des mesures différentielles réalisées avant et après dépôt de poly-L-lysine (figure 8A), réalisées en fonction de la concentration en KCl (figure 8B), et réalisées en fonction de la concentration en poly-L-lysine déposée.

A la figure 8A, les variations AI_{D} du courant de drain Id sont représentées en ordonnée pour chacun des transistors 60 à 96 identifiés en abscisse (U_{SG} = 1 V, U_{SD} = 0,9 V et électrolyte KCl à 0,1 mM). Les différences ΔI_{D} entre deux mesures réalisées avant un dépôt local mais séparées par un rinçage à l'eau sont représentées par des cercles. Les différences ΔI_{D} correspondant à des mesures effectuées avant et après un dépôt local de poly-L-lysine sont représentées par des étoiles. Après le dépôt local on laisse l'échantillon 15 minutes à température ambiante en milieu humide, avant de le sécher à 50°C pendant 5 minutes. La dilution Cₒ de la poly-L-lysine est de 0,01 % poids/volume "W/V" final (P8920, Sigma) dans un tampon PBS 0,1x à pH 7.

A la figure 8B, les différences ΔU_{SG} de la tension de source-grille U_{SG} sont mesurées sur une partie des transistors d'un réseau de 62 transistors FET avec U_{SD} = 1,2 V et I_{D} = 50 µA. Les différences entre une mesure de référence (effectuée avant dépôt local et avec une concentration en KCl de 0,01 mM) et de deux séries de mesures (effectués après dépôt local de poly-L-lysine et avec différentes concentrations en KCl) sont représentées par des cercles et des étoiles. Ici, un dépôt local de poly-L-lysine a été effectué dans deux zones distinctes avec la même dilution Cₒ que dans le cas de la figure 8A. Lors de chacune des deux séries de mesure, on fait varier la concentration de KCl dans le tampon de mesure entre 0,01 mM et 100 mM, en passant par les valeurs 0,1 mM, 1 mM et 10 mM . Entre les deux séries de mesure, on rince la surface à l'eau. On constate une sensibilité appréciable de la détection de la poly-L-lysine pour des concentrations de KCl entre 0,01 mM et 1 mM, et la hauteur des pics diminue progressivement au-delà de ces valeurs.

La figure 8C montre les variations ΔU_{SG} de la tension U_{SG} en fonction de la concentration en polymère déposé (poly-L-lysine), à savoir 2Cₒ, Cₒ, Cₒ/2, Cₒ/4, Cₒ/8 dans un tampon 0,1 x PBS pH 7, Cₒ ayant la valeur indiquée pour les mesures de la figure 8A. Les conditions de mesure sont les suivantes : U_{SD} = 1V, I_{D} = 100 µA, et une concentration de 0,01 mM pour KCl. Ces mesures montrent qu'il n'y a pas avantage dans les conditions expérimentales choisies d'augmenter la concentration au-delà de Cₒ.

Les figures 9A à 9D montrent la détection électronique d'ADN. Les tensions U_{SG} et les variations ΔU_{SG} de la tension U_{SG} correspondent à un point de fonctionnement U_{SD} = 1V, I_{D} = 100 µA, et à une concentration 0,01 mM en KCl. Elles sont obtenues à partir de la caractéristique I_{D}(U_{SG}, U_{SD}) et sont reportées sur les courbes avec en abscisse le numéro des transistors FET (1 à 96).

Les étoiles représentent la mesure avant traitement de surface initial à la soude tel qu'indiqué ci-dessus en liaison avec la figure 6. Les cercles représentent la mesure après incubation de poly-L-lysine sur tout le réseau. Pour permettre une immobilisation de l'ADN, on incube le réseau de transistors FET pendant 30 minutes dans une dilution de poly-L-lysine (concentration Co). Ensuite, sans effectuer de séchage préalable, on rince à l'eau et on sèche ensuite à l'air. L'incubation conduit à des décalages de la tension U_{SG} d'une valeur de 97 ± 50 mV (statistique sur 67 surfaces préparées) qui réduisent les variations entre transistors dans le signal électronique. Ces décalages sont compatibles à celles observées avec les valeurs mesurées en relation avec la figure 8C sur des dépôts locaux à la même concentration. Les carrés représentent les mesures après dépôt local d'oligonucléotides (5' Cy-5 modifiés 20 mer, concentration 50 µM dans de l'eau désionisée) autour des transistors n° 30, 60 et 90. En niveau de gris et au dessus de la figure 9A, est représentée l'image en microfluorescence des trois points d'ADN précités.

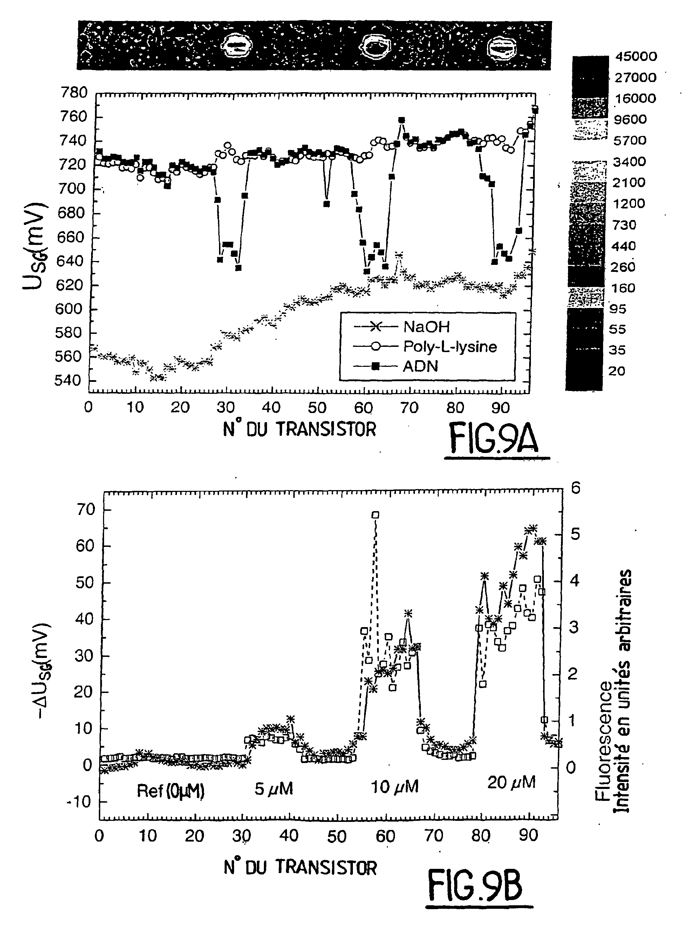

La figure 9B montre la détection électronique et par fluorescence d'oligonucléotides Cy5 modifiés. Les points représentés par des étoiles ont été obtenus par différence ΔU_{SG} entre deux mesures électroniques réalisées avant et après 4 dépôts locaux avec des concentrations différentes en ADN (Ref. = 0 µM, 5 µM 10 µM, 20 µM). Ils montrent la variation ΔU_{SG} de la tension U_{SG} qui est observée dans les caractéristiques des transistors et qui est due aux dépôts locaux d'ADN. Les carrés montrent l'intensité de la fluorescence mesurée sur les transistors FET séchés, une fois la mesure électronique réalisée avec l'électrolyte. On notera que la même détection électronique est obtenue avec des oligonucléotides du même type, mais non modifiés.

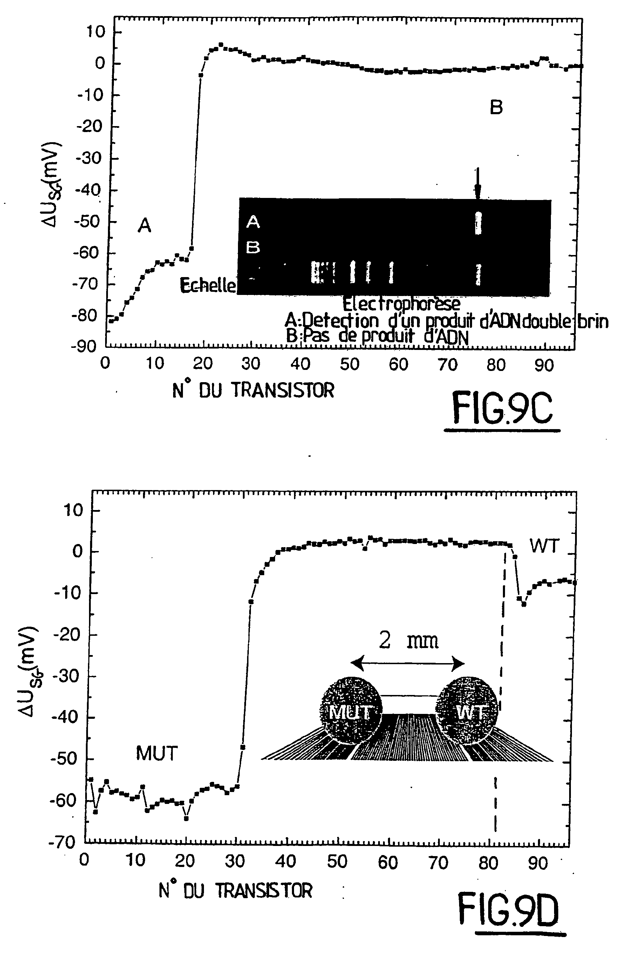

La figure 9C montre la détection d'ADN double-brin après dépôt macroscopique de deux produits sur deux zones A et B des transistors FET. Avec une micropipette, on dépose 0,15 µl prélevés dans deux tubes A et B sur deux régions respectives du réseau de transistors à effet de champ FET. Le réseau a été préalablement recouvert de poly-L-lysine pour immobiliser l'ADN et mesuré pour servir de référence. La zone A (transistors 1 à 20) de la figure 9C a été recouverte de la solution du tube A et la zone B (transistors 50 à 90) de la solution du tube B en laissant subsister entre elles une région centrale (transistors 21 à 49) non recouverte. On incube pendant 15 minutes sans sécher, puis on rince à l'eau et on mesure ensuite les transistors du réseau. Les transistors 1 à 20 (zone A) ont été incubés avec une solution contenant des produits d'une réaction par polymérisation en chaîne (PCR) obtenus dans le tube A selon la procédure décrite ci-après. On constate dans cette zone un décalage vers le bas par rapport à la zone B (transistors 50 à 90) et par rapport à ladite zone non incubée entre A et B. En effet, la solution de référence mise en oeuvre dans la zone B a été choisie pour ne pas produire de l'ADN double brin (tube B selon la procédure décrite ci-après).

A la figure 9D, dans la région MUT (transistors 1 à 35) sur laquelle a été déposée une solution obtenue par amplification PCR dans des conditions décrites ci-après, à partir d'ADN portant une mutation, on observe un décalage vers le bas de ΔU_{SG}, alors que dans la région de référence WT, pour laquelle l'ADN de départ ne portant pas la mutation, on n'observe pas un tel décalage.

Pour l'expérience de la figure 9C, la technique d'amplification PCR d'un fragment d'ADN à 1009 paires de bases met en oeuvre l'ADN du bactériophage λ digéré par l'enzyme BstEll en utilisant deux amorces:
5'- CCG CGA ACT GAC TCT CCG CC
et 5' -CAG GCG GCA GGG CTG ACG TT .

Le protocole PCR est conduit sur un thermocycleur du commerce :
- initiation pendant 3 minutes à 94 °C
- 30 cycles de dénaturation / hybridation / extension de 30 secondes à 94°C/30 secondes à 57°C / et 2 minutes à 72°C.

Une étape finale de PCR est conduite pendant 3 minutes à 72°C.

Pour un volume de 50 microlitres, on utilise 10 nanogrammes de l'ADN λ digéré par Bst EII, 20 picomoles pour chacune des amorces, et les quatre dNTP ont une concentration finale de 50 µM chaque. On met 0,5 microlitres de polymérase TAQ (à 1U/ µl) de Roche Diagnostics dans le tampon standard de réaction PCR (fourni avec la polymérase TAQ). Ceci correspond à la préparation du tube A pour la zone A. Dans le tube de référence B (qui correspond à la zone B), on remplace un des quatre dNTP, à savoir le dTTP par du dCTP, de manière à conserver la même concentration totale en dNTP, ce qui inhibe la synthèse du produit d'ADN double brin.

Les produits de la PCR sont, dans l'un et l'autre cas, purifiés deux fois sur des colonnes "QIAQUICK" de la Société QIAGEN et éluées avec un tampon de Tris-Cl de pH 8,5 à une concentration de 10 mM.

L'amplification PCR spécifique de la mutation mise en oeuvre dans le cadre de l'expérience correspondant à la figure 9D part d'un fragment du gène humain CX-26 (code d'accès M 86849, chromosome 13q11-12). Ce gène est amplifié à partir d'ADN génomique provenant d'un ou plusieurs patients. La technique de PCR met en oeuvre des conditions et des amorces de cyclage décrites dans les articles de F. DENOYELLE et collaborateurs utilisés pour le premier "Prelingual Deafness : hight prevalence of a 30delG mutation in the connexin 26 gene" paru dans Human Molecular Genetics, 1997, vol. 6, n° 12, p. 2173 à 2177 et pour le deuxième "Clinical features of the prevalent form of childhood deafness, DFNB1, due to a connexin-26 gene defect : implications for genetic counselling" paru dans THE LANCET, vol. 353, 17 Avril 1999, p. 1298 à 1303. On utilise une polymèrase Pwo (de Roche Diagnostics) dans un tampon PCR avec MgSO₄ à 1,5 mM. Les amorces sont le GAP1F et CONNR (voir le deuxième article précité de F. DENOYELLE, p. 1299 colonne de droite avant dernier paragraphe) et les conditions expérimentales sont celles du premier article précité du même auteur (p. 2177). On utilise une concentration finale de 0, 6 µM pour chacune des amorces et de 0,2 mM pour chacun des dNTP.

Les produits de la PCR sont purifiés sur des colonnes "QIAQUICK" de la Société QIAGEN, et après une dilution (de 10 000 fois) servent d'ADN de départ dans la réaction spécifique de la mutation qui a lieu ensuite.

L'amplification PCR est choisie pour permettre la détection de la mutation 35delG (ou 30delG) dans le gène CX26, grâce à des amorces spécifiques de cette mutation. Les conditions de cycle et les séquences des amorces sont données dans l'article de G. LUCOTTE et collaborateurs intitulé "PCR test for diagnosis of the common GJB2 (connexin 26) 35delG mutation on dried blood spots and determination of the carrier frequency in France" publié dans Molecular and Cellular Probes (2001) 15, p. 57 à 59. On utilise 20 picomoles de chacun des oligonucléotides amorce pour un volume final de 50 µl.

Les deux amorces spécifiques de la mutation (voir article de LUCOTTE précité, page 58, colonne de droite M primer et N primer) et une amorce commune (C primer) sont utilisées pour synthétiser les produits de PCR de 197 paires de bases. On réalise deux réactions PCR spécifiques sur chaque échantillon d'ADN, la première de ces réactions est réalisée avec la première amorce spécifique et donne un produit si la mutation est présente dans l'ADN de départ. La deuxième réaction est réalisée avec la deuxième amorce spécifique et donne un produit si la mutation n'est pas présente dans l'ADN de départ. Ceci permet de déterminer si un échantillon est normal, hétérozygote ou homozygote vis-à-vis de cette mutation.

Pour un volume de 50 µl de milieu réactif, dans un tampon PCR standard, on utilise un microlitre d'ADN provenant de la préamplification décrite ci-dessus, 30 picomoles de chacune des amorces, des dTNP à une teneur 100 µM et 1 microlitre de polymérase TAQ (1 U/ µl) de Roche Diagnosis. Les produits de PCR sont purifiés deux fois sur des colonnes "QIAQUICK" de la Société QIAGEN, et éluées avec un tampon Tris-Cl à 10 mM de pH 8,5. Pour les tube WT et MUT, on a utilisé la même paire d'amorces : C-primer et M-primer. La seule différence est l'ADN de départ.

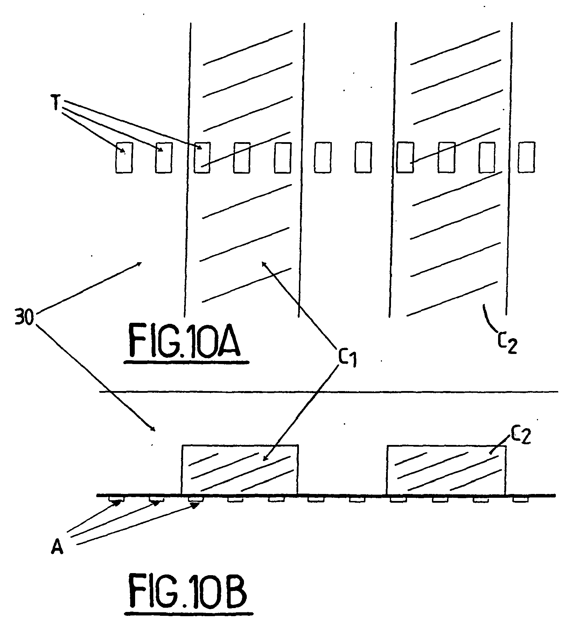

Les figures 10A et 10B montrent un circuit intégré présentant des transistors T arrangés selon une ligne (ou plusieurs lignes). Deux canaux microfluidiques (par exemple parallèles) C₁ et C₂ d'un substrat 30 permettent de mettre un ou plusieurs transistors T à effet de champ en contact avec la solution qui circule dans un canal C₁ et/ou C₂. Le matériau d'un substrat 30 qui compporte les canaux microfluidiques (ou capillaires) peut être un polymère PDMS (polydiméthylsiloxane) ou autre, un verre, du silicium, etc...

Il est ainsi possible de réaliser des mesures différentielles à partir de deux solutions qui circulent dans les deux canaux C₁ et C₂. Il est également possible de réaliser sur un même substrat 30 un grand nombre de tels micro-canaux fluidiques, le substrat dans lequel ils sont ménagés étant solidarisé au substrat semi-conducteur dans lequel sont intégrés les transistors à effet de champ FET. Il est également possible de mesurer une variation à l'intérieur d'un canal donné. Cette variation peut être au cours du temps. Il est également possible d'injecter différentes solutions à un endroit donné dans un capillaire et le profil des concentrations reste inchangé le long du canal, même loin du point d'injection. On se reportera à l'article de Paul J. A. KENIS et al., intitulé "Microfabrication inside Capillaries Using Multiphase Laminar Flow Patteming" paru dans SCIENCE, vol. 285, 2 Juillet 1999, p. 83-85 (notamment figure 1A).

Une technique d'analyse mettant en oeuvre la microfluidique est décrite dans l'article "Monolithic integrated microfluidic DNA amplification and capillary electrophoresis analysis system" de Eric T. LAGALLY et collaborateurs paru dans Sensors and Actuators B 63 (2000) p. 138-146.

## Revendications

1. Procédé de détection de la fixation de molécules sondes sur des zones actives d'un capteur ou d'une interaction, **caractérisé en ce que** ledit capteur est constitué par un réseau de transistors à effet de champ (T₁, T₂ ...) dont chacun présente une région de source (S), une région de drain (D), ainsi qu'une région de porte qui constitue une dite zone active (3) sur laquelle ladite fixation ou ladite interaction doit être détectée en fixant le potentiel d'une solution de mesure qui recouvre lesdites zones actives (3) grâce à une électrode de porte immergée dans ladite solution de mesure (E) et **en ce qu'**il comporte les étapes suivantes :
a) mettre en contact certaines desdites zones (3) avec des molécules sondes pour réaliser leur fixation,
b) baigner dans une solution d'électrolyte (6) au moins ces zones qui ont été mises en contact avec des molécules sondes,
c) mesurer au moins un point de la caractéristique courant de drain/tension source-porte/tension source-drain d'au moins deux des transistors d'un premier groupe correspondant à au moins une zone active (3) mise en contact avec des molécules sondes pour en déduire une dite fixation ou une dite interaction par comparaison entre au moins deux dites mesures obtenues pour deux différentes zones.

2. Procédé selon la revendication 1, **caractérisé en ce que** ladite mesure d'au moins un point de la caractéristique met en oeuvre l'application d'une tension donnée (U_{DS}) entre le drain et la source d'au moins lesdits deux transistors du premier groupe ainsi que l'application d'une tension donnée (U_{GS}) entre la porte et la source de ces transistors du premier groupe.

3. Procédé selon une des revendications précédentes, **caractérisé en ce qu'**il présente entre a et b une étape de rinçage.

4. Procédé selon une des revendications précédentes, **caractérisé en ce qu'**il comporte après a) et avant b) les étapes suivantes:
a1) rinçage
a2) ajout d'une solution contenant des molécules cibles susceptibles d'interagir spécifiquement avec les molécules sondes.

5. Procédé selon une des revendications précédentes, **caractérisé en ce qu'**il comporte après c, les étapes suivantes :
d) ajout d'une solution d'électrolyte (6) contenant des molécules cibles susceptibles d'interagir spécifiquement avec les molécules sondes.
e) mesurer au moins un point de la caractéristique courant de drain/tension, source-porte / tension source-drain d'au moins deux des transistors d'un deuxième groupe correspondant à au moins une zone active (3) mise en contact avec des molécules sondes et avec des molécules cibles pour obtenir par comparaison une détection d'au moins une interaction.

6. Procédé selon la revendication 5, **caractérisé en ce que**, au point e, la mesure d'au moins un point de la caractéristique met en oeuvre l'application d'une tension donnée (U_{DS}) entre le drain et la source des transistors d'au moins lesdits deux transistors du deuxième groupe, et l'application d'une tension donnée (U_{GS}) entre la porte et la source de ces transistors du deuxième groupe.

7. Procédé selon une des revendications 5 ou 6, **caractérisé en ce qu'**il met en oeuvre une pluralité de dites mesures d'au moins un point de la caractéristique, qui sont espacées au cours du temps.

8. Procédé selon une des revendications précédentes, **caractérisé en ce que** ladite comparaison est réalisée par mesure différentielle.

9. Procédé selon une des revendications précédentes, **caractérisé en ce que** la comparaison est réalisée entre des mesures effectuées sur au moins deux transistors correspondant à des zones (3) qui sont baignées par une solution d'électrolyte (6) après avoir été mises en contact avec des molécules sondes.

10. Procédé selon une des revendications 1 à 8, **caractérisé en ce que** la comparaison est réalisée entre des mesures effectuées sur au moins un transistor correspondant à une zone (3) qui est baignée par une solution d'électrolyte (6) après avoir été mise en contact avec des molécules sondes en vue de leur fixation et sur au moins un transistor correspondant à une zone qui est baignée par ladite solution d'électrolyte (6) sans avoir été mise en contact avec des molécules sondes.

11. Procédé selon une des revendications précédentes, **caractérisé en ce que** les molécules sondes sont des molécules d'ADN, d'ARN ou de protéines.

12. Procédé selon la revendication 11, **caractérisé en ce que** les molécules sondes sont des molécules d'ADN et **en ce que** les transistors à effet de champ sont de type à canal n à appauvrissement, avec une polarisation de porte négative.

13. Procédé selon une des revendications 11 ou 12, **caractérisé en ce qu'**il met en oeuvre une détection par comparaison entre deux zones comprenant chacune au moins un dit transistor à effet de champ, la première zone étant baignée par une solution obtenue à partir d'une réaction enzymologique donnant un produit détectable spécifique de la présence ou de l'absence d'une mutation dans un premier échantillon d'ADN et la deuxième zone étant baignée par une solution obtenue à partir d'une réaction enzymologique donnant un produit d'ADN spécifique de la présence ou de l'absence d'une mutation dans un deuxième échantillon d'ADN.

14. Procédé selon la revendication 13, **caractérisé en ce que** le premier et le deuxième échantillons d'ADN proviennent de deux patients et **en ce que** la réaction enzymologique est la même pour les deux échantillons.

15. Procédé selon la revendication 13, **caractérisé en ce que** le premier et le deuxième échantillons d'ADN sont identiques et proviennent du même patient et **en ce que** la réaction enzymologique dans la première zone est réalisée dans des conditions expérimentales produisant un produit d'ADN en l'absence de mutation dans le premier échantillon et **en ce que** la réaction enzymologique dans la deuxième zone est réalisée dans des conditions expérimentales produisant un produit d'ADN en présence d'une mutation dans le deuxième échantillon.

16. Procédé selon une des revendications précédentes, **caractérisé en ce qu'**il comporte la circulation à travers au moins un microcanal fluidique d'une solution pour la mettre en contact avec au moins un dit transistor à effet de champ (T₁, T₂, ...).

## Claims

1. A method for detecting the fixing of molecular probes to active zones of a sensor or an interaction, **characterized in that** said sensor consists of a network of field-effect transistors (T₁, T₂ etc.), each of which has a source region (S), a drain region (D), and a gate region which forms a said active zone (3) on which said fixing or said interaction should be detected by fixing the potential of a measurement solution which covers said active zones (3) by means of a gate electrode immerged in said measurement solution (E), and **in that** it comprises the following steps:
a) bringing some of said zones (3) into contact with molecular probes in order to fix said probes,
b) bathing at least these zones which have been brought into contact with molecular probes, in an electrolyte solution (6),
c) measuring at least one point of the drain current/source-gate voltage/source-drain voltage characteristic of at least two of the transistors of a first group corresponding to at least an active zone (3) brought into contact with molecular probes, so as to deduce therefrom at least one said fixing or one said interaction by comparison between at least two measurements obtained for two different zones.

2. The method as claimed in claim 1, **characterized in that** said measurement of at least one point of the characteristic uses the application of a given voltage (U_{DS}) between the drain and the source of at least said two transistors of the first group and also the application of a given voltage (U_{GS}) between the gate and the source of these transistors of the first group.

3. The method as claimed in either of the preceding claims, **characterized in that** it has a rinsing step between a and b.

4. The method as claimed in one of the preceding claims, **characterized in that** it comprises, after a) and before b), the following steps:
a1) rinsing,
a2) adding a solution containing target molecules capable of interacting specifically with the molecular probes.

5. The method as claimed in one of the preceding claims, **characterized in that** it comprises, after c, the following steps:
d) adding an electrolyte solution (6) containing target molecules capable of interacting specifically with the molecular probes,
e) measuring at least one point of the drain current/source-gate voltage/source-drain voltage characteristic of at least two of the transistors of a second group corresponding to at least an active zone (3) brought into contact with molecular probes and with target molecules, so as to obtain by comparison a detection of at least an interaction.

6. The method as claimed in claim 5, **characterized in that**, in point e, the measurement of at least one point of the characteristic uses the application of a given voltage (U_{DS}) between the drain and the source of the transistors of at least said two transistors of the second group, and the application, of a given voltage (U_{GS}) between the gate and the source of these transistors of the second group.

7. The method as claimed in either of claims 5 and 6, **characterized in that** it uses a plurality of said measurements of at least one point of the characteristic, which are spaced out over time.

8. The method as claimed in one of the preceding claims, **characterized in that** said comparison is carried out by differential measurement.

9. The method as claimed in one of the preceding claims, **characterized in that** the comparison is carried out between measurements carried out on at least two transistors corresponding to zones (3) which are bathed in an electrolyte solution (6) after having been brought into contact with molecular probes.

10. The method as claimed in one of claims 1 to 8, **characterized in that** the comparison is carried out between measurements carried out on at least one transistor corresponding to a zone (3) which is bathed in an electrolyte solution (6) after having been brought into contact with molecular probes for the purpose of fixing them, and on at least one transistor corresponding to a zone which is bathed in said electrolyte solution (6) without having been brought into contact with molecular probes.

11. The method as claimed in one of the preceding claims, **characterized in that** the molecular probes are DNA, RNA or protein molecules.

12. The method as claimed in claim 11, **characterized in that** the molecular probes are DNA molecules and **in that** the field-effect transistors are of depleted n-channel type, with a negative gate bias.

13. The method as claimed in either of claims 11 and 12, **characterized in that** it uses detection by comparison between two zones, each comprising at least one said field-effect transistor, the first zone being bathed in a solution obtained from an enzymological reaction giving a detectable product specific for the presence or the absence of a mutation in a first DNA sample, and the second zone being bathed in a solution obtained from an enzymological reaction giving a DNA product specific for the presence or for the absence of a mutation in a second DNA sample.

14. The method as claimed in claim 13, **characterized in that** the first and the second DNA samples originate from two patients and **in that** the enzymological reaction is the same for the two samples.

15. The method as claimed in claim 13, **characterized in that** the first and the second DNA samples are identical and originate from the same patient, and **in that** the enzymological reaction in the first zone is carried out under experimental conditions producing a DNA product in the absence of mutation in the first sample, and **in that** the enzymological reaction in the second zone is carried out under experimental conditions producing a DNA product in the presence of a mutation in the second sample.

16. The method as claimed in one of the preceding claims, **characterized in that** it comprises the circulation of a solution through at least one microfluid channel, so as to bring it into contact with at least one said field-effect transistor (T₁ ... T₂ etc.).

## Patentansprüche

1. Verfahren zum Erfassen der Fixierung von Sondenmolekülen auf aktiven Zonen eines Sensors oder einer Wechselwirkung, **dadurch gekennzeichnet, dass** der Sensor aus einem Netzwerk von Feldeffekttransistoren (T₁, T₂, ...) besteht, mit jeweils einer Source-Zone (S), einer Drain-Zone (D), sowie einer Gate-Zone, welche eine besagte aktive Zone (3) darstellt, auf der die Fixierung oder die Wechselwirkung erfasst werden soll, indem das Potential einer Messlösung, welche die aktiven Zonen (3) bedeckt, mithilfe einer in die Messlösung (E) eingetauchten Gate-Elektrode festgelegt wird, sowie **dadurch**, dass das Verfahren die folgenden Schritte umfasst:
a) In-Kontakt-Bringen von bestimmten der Zonen (3) mit Sondenmolekülen, um deren Fixierung zu bewirken,
b) Baden zumindest derjenigen Zonen, die mit Sondenmolekülen in Kontakt gebracht wurden, in einer Elektrolytlösung (6),
c) Messen von mindestens einem Punkt der Drain-Strom/Source-Gate-Spannung/Source-Drain-Spannung-Kennlinie von mindestens zwei der Transistoren einer ersten Gruppe, die mindestens einer aktiven Zone (3) entspricht, welche mit Sondenmolekülen in Kontakt gebracht wurde, um daraus mittels Vergleich zwischen mindestens zwei solchen Messungen, die für zwei verschiedene Zonen erhalten wurden, eine Fixierung oder eine Wechselwirkung abzuleiten.

2. Verfahren nach Anspruch 1, **dadurch gekennzeichnet, dass** das Messen von mindestens einem Punkt der Kennlinie das Anlegen einer gegebenen Spannung (U_{DS}) zwischen dem Drain und der Source von zumindest den zwei Transistoren der ersten Gruppe sowie das Anlegen einer gegebenen Spannung (U_{GS}) zwischen dem Gate und der Source dieser Transistoren der ersten Gruppe anwendet.

3. Verfahren nach einem der vorherigen Ansprüche, **dadurch gekennzeichnet, dass** es zwischen a) und b) einen Spülschritt umfasst.

4. Verfahren nach einem der vorherigen Ansprüche, **dadurch gekennzeichnet, dass** es nach a) und vor b) die folgenden Schritte umfasst:
a1) Spülen
a2) Zugeben einer Lösung, welche Zielmoleküle enthält, die in der Lage sind, spezifisch mit den Sondenmolekülen eine Wechselwirkung einzugehen.

5. Verfahren nach einem der vorheringen Ansprüche, **dadurch gekennzeichnet, dass** es nach c) die folgenden Schritte umfasst:
d) Zugeben einer Elektrolytlösung (6), welche Zielmoleküle enthält, die in der Lage sind, spezifisch mit den Sondenmolekülen eine Wechselwirkung einzugehen.
e) Messen von mindestens einem Punkt der Drain-Strom/Source-Gate-Spannung/Source-Drain-Spannung-Kennlinie von mindestens zwei der Transistoren einer zweiten Gruppe, die mindestens einer aktiven Zone (3) entspricht, welche mit Sondenmolekülen und mit Zielmolekülen in Kontakt gebracht wurde, um mittels Vergleich eine Erfassung von zumindest einer Wechselwirkung zu erhalten.

6. Verfahren nach Anspruch 5, **dadurch gekennzeichnet, dass** bei e) die Messung von mindestens einem Punkt der Kennlinie das Anlegen einer gegebenen Spannung (U_{DS}) zwischen dem Drain und der Source der Transistoren von zumindest den zwei Transistoren der zweiten Gruppe und das Anlegen einer gegebenen Spannung (U_{GS}) zwischen dem Gate und der Source dieser Transistoren der zweiten Gruppe umfasst.

7. Verfahren nach einem der Ansprüche 5 oder 6, **dadurch gekennzeichnet, dass** es eine Mehrzahl solcher Messungen von mindestens einem Punkt der Kennlinie anwendet, die zeitlich beabstandet sind.

8. Verfahren nach einem der vorherigen Ansprüche, **dadurch gekennzeichnet, dass** der Vergleich mittels differentieller Messung durchgeführt wird.

9. Verfahren nach einem der vorherigen Ansprüche, **dadurch gekennzeichnet, dass** der Vergleich zwischen Messungen durchgeführt wird, die an mindestens zwei Transistoren vorgenommen werden, welche Zonen (3) entsprechen, die in einer Elektrolytlösung (6) gebadet sind, nachdem sie mit Sondenmolekülen in Kontakt gebracht wurden.

10. Verfahren nach einem der Ansprüche 1 bis 8, **dadurch gekennzeichnet, dass** der Vergleich zwischen Messungen durchgeführt wird, die an mindestens einem Transistor, welcher einer Zone (3) entspricht, die in einer Elektrolytlösung (6) gebadet ist, nachdem sie mit Sondenmolekülen in Kontakt gebracht wurde, um diese zu fixieren, sowie an mindestens einem Transistor, welcher einer Zone entspricht, die von der Elektrolytlösung (6) gebadet ist, ohne mit Sondenmolekülen in Kontakt gebracht worden zu sein, vorgenommen werden.

11. Verfahren nach einem der vorherigen Ansprüche, **dadurch gekennzeichnet, dass** die Sondenmoleküle DNA-, RNA- oder Proteinmoleküle sind.

12. Verfahren nach Anspruch 11, **dadurch gekennzeichnet, dass** die Sondenmoleküle DNA-Moleküle sind, sowie **dadurch**, dass die Feldeffekttransistoren vom N-Kanal-Verarmungstyp mit einer negativen Gate-Vorspannung sind.

13. Verfahren nach einem der Ansprüche 11 oder 12, **dadurch gekennzeichnet, dass** es eine Erfassung mittels Vergleich zwischen zwei Zonen anwendet, die jeweils mindestens einen besagten Feldeffekttransistor aufweisen, wobei die erste Zone von einer Lösung gebadet ist, die ausgehend von einer enzymologischen Reaktion erhalten wird, welche ein erfassungsfähiges Produkt ergibt, das für das Vorliegen oder das Nichtvorliegen einer Mutation in einer ersten DNA-Probe spezifisch ist, und die zweite Zone von einer Lösung gebadet ist, die ausgehend von einer enzymologischen Reaktion erhalten wird, welche ein DNA-Produkt ergibt, das für das Vorliegen oder das Nichtvorliegen einer Mutation in einer zweiten DNA-Probe spezifisch ist.

14. Verfahren nach Anspruch 13, **dadurch gekennzeichnet, dass** die erste und die zweite DNA-Probe von zwei Patienten stammen, sowie **dadurch**, dass die enzymologische Reaktion bei den beiden Proben die gleiche ist.

15. Verfahren nach Anspruch 13, **dadurch gekennzeichnet, dass** die erste und die zweite DNA-Probe identisch sind und von dem gleichen Patienten stammen, sowie **dadurch**, dass die enzymologische Reaktion in der ersten Zone unter experimentellen Bedingungen durchgeführt wird, welche bei Nichtvorliegen einer Mutation in der ersten Probe ein DNA-Produkt ergeben, sowie **dadurch**, dass die enzymologische Reaktion in der zweiten Zone unter experimentellen Bedingungen durchgeführt wird, welche bei Vorliegen einer Mutation in der zweiten Probe ein DNA-Produkt ergeben.

16. Verfahren nach einem der vorherigen Ansprüche, **dadurch gekennzeichnet, dass** es das Zirkulieren einer Lösung durch mindestens eine Mikrofluidpassage umfasst, um die Lösung mit mindestens einem besagten Feldeffekttransistor (T₁, T₂, ...) in Kontakt zu bringen.
